# EUROPEAN PATENT APPLICATION

(11) **EP 3 058 939 A1**
(43) Date of publication of application: **24.08.2016**
(21) Application number: 14854118.8
(22) Date of filing: 17.10.2014
(51) Int. Cl.: A61K 31/352, A61P 33/10

(54) **ANTIPARASITIC AGENT**

(30) Priority: 18.10.2013 JP 2013216996
(71) Applicant: NAI Inc., Minato-ku Tokyo 107-0061 (JP)
(72) Inventor: KITA, Kiyoshi, Tokyo 171-0031 (JP); INAOKA, Ken Daniel, Tokyo 140-0014 (JP); YAMAMOTO, Masaichi, Tokyo 107-0061 (JP)
(74) Representative: Michalík, Andrej
(86) International application number: PCT/JP2014/077638
(87) International publication number: WO 2015/056768

(57) **Abstract**

Provided is a new antiparasitic agent for medical use, veterinary use, agrochemical use, and use in fish, said antiparasitic agent having an effect on both helminths and protozoa. The antiparasitic agent uses at least one siccanin produced by *Helminthosproium siccans* which is a filamentous fungus, a synthetic siccanin, or a modified derivative thereof.

## Description

### TECHNICAL FIELD

The present invention provides a prophylactic and therapeutic agent for parasitic infections in a wide range of organisms such as humans, animals other than humans, and fish, comprising at least one compound selected from a siccanin and a modified derivative thereof as an active ingredient (active compound).

That is, the present invention relates to an antiparasitic agent comprising as an active ingredient a siccanin produced by *Helminthosporium siccans* which is a filamentous fungus, a synthetic siccanin, or a modified derivative thereof.

### BACKGROUND ART

Parasitic diseases are classified into two categories, helminthic diseases which are caused by multicellular helminths and protozoan diseases which are caused by unicellular protozoa, each type causing significant health damage chiefly in developing countries.

Helminthic diseases are caused by helminths which are eukaryotic invertebrates, and are classified into cestode infection, animal helminthic disease, nematode infection, and trematode infection.

Among helminthic diseases, soil-transmitted helminth infections are one of those which affect the greatest number of individuals and are estimated to infect 1.5 billion people chiefly in Asia, Africa, and Latin America, annually killing 130,000 people, especially children (WHO 2011). Most of soil-transmitted helminth infections are caused by roundworms, which infect 1.2 billion people, chiefly in Asia. Ascariasis may cause diarrhea, stomachache, and malnutrition or disturbance of development in infants, sometimes resulting in deaths, and livestock infections also cause significant economic damage. Currently, levamisole, pyrantel, albendazole and mebendazole (benzimidazoles-based drugs), as well as avermectin and milbemycin which are macrocyclic lactones are known as therapeutic agents for helminthic disease. However, no novel antiparasitic agent has been commercialized since 1970s, so with concerns over drug resistance, a need exists for the development of a novel agent.

On the other hand, protozoan diseases are caused by infection with protozoa which are unicellular and eukaryotic organisms and morphologically, they are classified into mastigophora, sporozoa, rhizopoda, and ciliates.

Protozoan diseases including malaria and trypanosomiasis cause significant health damage. Malaria is caused by *Plasmodium spp.* infection and estimated to affect 216 million people in 195 nations in the world, killing 665 thousand people annually. As antimalarial agents, chloroquine, mefloquine, sulfadoxin, and artemisinin have heretofore been developed, but with the reporting of emergence of resistant strains for all of these agents (Non-Patent Documents 1 and 2), concurrent administration is a treatment of first choice. Trypanosomiasis is caused by trypanosomatids, and, in particular, African trypanosomiasis caused by *Trypanosoma brucei*, Chagas disease by *T. cruzi*, and leishmaniasis by *Leishmania spp.* are called three major types of trypanosomiasis. It is estimated that in 2009, about 30 thousand people, mainly in Africa, were annually infected with African sleeping sickness (WHO, 2009) whereas 10 million people, mainly in Latin America, were infected with Chagas disease (WHO, 2010). Leishmaniasis, classified into cutaneous leishmaniasis, mucocutaneous leishmaniasis, and visceral leishmaniasis, is estimated to infect 350 million people, mainly in Southeast Asia and Latin America. Among the aforementioned diseases, African trypanosomiasis, visceral leishmaniasis, and chronic Chagas disease may result in death unless adequate treatment is given. Therapeutic agents currently used for such diseases include benznidazole, suramin, melarsoprol, nifurtimox, and eflornithine for African sleeping sickness (Non-Patent Document 3); benznidazole and nifurtimox for Chagas disease (non-patent document 4); and Glucantime, Pentaptom, amphotericin B, miltefosine, and Paromomycin for leishmaniasis (Non-Patent Document 5). However, most of these therapeutic agents have serious adverse effects and, in addition, their efficacy may be low depending on the stage in the progression of the diseases, with the result that drug resistant strains have begun to emerge in recent years (Non-Patent Documents 6 and 7).

### CITATION LIST

### NON-PATENT DOCUMENTS

[Non-Patent Document 1] Mita, T., Tanabe, K., and Kita, K. (2009). Spread and evolution of Plasmodium falciparum drug resistance. Parasitol Int 58, 201-209.
[Non-Patent Document 2] Denis, M.B., Tsuyuoka, R., Lim, P., Lindegardh, N., Yi, P., Top, S.N., Socheat, D., Fandeur, T., Annerberg, A., Christophel, E.M., et al. (2006). Efficacy of artemether-lumefantrine for the treatment of uncomplicated falciparum malaria in northwest Cambodia. Trop Med Int Health 11, 1800-1807.
[Non-Patent Document 3] Brun, R., Blum, J., Chappuis, F., and Burri, C. (2010). Human African trypanosomiasis. Lancet 375, 148-159.
[Non-Patent Document 4] Rassi, A., and Marin-Neto, J.A. (2010). Chagas disease. Lancet 375, 1388-1402.
[Non-Patent Document 5] Singh, N., Kumar, M., and Singh, R.K. (2012). Leishmaniasis: current status of available drugs and new potential drug targets. Asian Pac J Trop Med 5, 485-497.
[Non-Patent Document 6] Chakravarty, J., and Sundar, S. (2010). Drug resistance in leishmaniasis. J Glob Infect Dis 2, 167-176.
[Non-Patent Document 7] Baker, N., de Koning, H.P., Maser, P., and Horn, D. (2013). Drug resistance in African trypanosomiasis: the melarsoprol and pentamidine story. Trends Parasitol 29, 110-118.
[Non-Patent Document 8] Harada, S., Inaoka, D.K., Ohmori, J., and Kita, K. (2013). Diversity of parasite complex II. Biochim Biophys Acta 1827, 658-667.
[Non-Patent Document 9] Cecchini, G. (2003). Function and structure of complex II of the respiratory chain. Annu Rev Biochem 72, 77-109.
[Non-Patent Document 10] Pereira, M.M., Carita, J.N., and Teixeira, M. (1999). Membrane-bound electron transfer chain of the thermohalophilic bacterium Rhodothermus marinus: characterization of the iron-sulfur centers from the dehydrogenases and investigation of the high-potential iron-sulfur protein function by in vitro reconstitution of the respiratory chain. Biochemistry 38, 1276-1283.
[Non-Patent Document 11] Millar, A.H., Eubel, H., Jänsch, L., Kruft, V., Heazlewood, J.L., and Braun, H.P. (2004). Mitochondrial cytochrome c oxidase and succinate dehydrogenase complexes contain plant specific subunits. Plant Mol Biol 56, 77-90.
[Non-Patent Document 12] Morales, J., Mogi, T., Mineki, S., Takashima, E., Mineki, R., Hirawake, H., Sakamoto, K., Omura, S., and Kita, K. (2009). Novel mitochondrial complex II isolated from Trypanosoma cruzi is composed of 12 peptides including a heterodimeric Ip subunit. J Biol Chem 284, 7255-7263.
[Non-Patent Document 13] Luque-Ortega, J.R., and Rivas, L. (2007). Miltefosine (hexadecylphosphocholine) inhibits cytochrome c oxidase in Leishmania donovani promastigotes. Antimicrob Agents Chemother 51, 1327-1332.
[Non-Patent Document 14] Carvalho, L., Luque-Ortega, J.R., Manzano, J.I., Castanys, S., Rivas, L., and Gamarro, F. (2010). Tafenoquine, an antiplasmodial 8-aminoquinoline, targets leishmania respiratory complex III and induces apoptosis. Antimicrob Agents Chemother 54, 5344-5351.
[Non-Patent Document 15] Carvalho, L., Luque-Ortega, J.R., López-Martín, C., Castanys, S., Rivas, L., and Gamarro, F. (2011). The 8-aminoquinoline analogue sitamaquine causes oxidative stress in Leishmania donovani promastigotes by targeting succinate dehydrogenase. Antimicrob Agents Chemother 55, 4204-4210.
[Non-Patent Document 16] Ishibasi, K. (1962). Studies on antibiotics from Helminthosporium sp. fungi .7. siccanin, a new antifungal antibiotic produced by Helminthosporium siccans. Journal of Antibiotics 15, 161-&.
[Non-Patent Document 17] Hirai, K., Okuda, S., Nozoe, S., and Iitaka, Y. (1969). The crystal and molecular structure of siccanin p-bromobenzenesulphonate. Acta Crystallogr B 25, 2630-2638.
[Non-Patent Document 18] Arai, M., Ishibashi, K., and Okazaki, H. (1969). Siccanin, a new antifungal antibiotic. I. In vitro studies. Antimicrob Agents Chemother (Bethesda) 9, 247-252.
[Non-Patent Document 19] Sugawara, S. (1969). Siccanin, a new antifungal antibiotic. II. In vivo studies. Antimicrob Agents Chemother (Bethesda) 9, 253-256.
[Non-Patent Document 20] Nose, K., and Endo, A. (1971). Mode of action of the antibiotic siccanin on intact cells and mitochondria of Trichophyton mentagrophytes . J Bacteriol 105, 176-184.
[Non-Patent Document 21] Mogi, T., Kawakami, T., Arai, H., Igarashi, Y., Matsushita, K., Mori, M., Shiomi, K., Omura, S., Harada, S., and Kita, K. (2009). Siccanin rediscovered as a species-selective succinate dehydrogenase inhibitor. J Biochem 146, 383-387.
[Non-Patent Document 22] Osanai, A., Harada, S., Sakamoto, K., Shimizu, H., Inaoka, D.K., and Kita, K. (2009). Crystallization of mitochondrial rhodoquinol-fumarate reductase from the parasitic nematode Ascaris suum with the specific inhibitor flutolanil. Acta Crystallogr Sect F Struct Biol Cryst Commun 65, 941-944.
[Non-Patent Document 23] Shimizu, H., Osanai, A., Sakamoto, K., Inaoka, D.K., Shiba, T., Harada, S., and Kita, K. (2012). Crystal structure of mitochondrial quinol-fumarate reductase from the parasitic nematode Ascaris suum. J Biochem 151, 589-592.
[Non-Patent Document 24] Sun, F., Huo, X., Zhai, Y., Wang, A., Xu, J., Su, D., Bartlam, M., and Rao, Z. (2005). Crystal structure of mitochondrial respiratory membrane protein complex II. Cell 121, 1043-1057.
[Non-Patent Document 25] Escobar, P., Matu, S., Marques, C., and Croft, S.L. (2002). Sensitivities of Leishmania species to hexadecylphosphocholine (miltefosine), ET-18-OCH(3) (edelfosine) and amphotericin B. Acta Trop 81, 151-157.
[Non-Patent Document 26] Luna, K.P., Hernández, I.P., Rueda, C.M., Zorro, M.M., Croft, S.L., and Escobar, P. (2009). In vitro susceptibility of Trypanosoma cruzi strains from Santander, Colombia, to hexadecylphosphocholine (miltefosine), nifurtimox and benznidazole. Biomedica 29, 448-455.
[Non-Patent Document 27] Räz, B., Iten, M., Grether-Bühler, Y., Kaminsky, R., and Brun, R. (1997). The Alamar Blue assay to determine drug sensitivity of African trypanosomes (T.b. rhodesiense and T.b. gambiense) in vitro. Acta Trop 68, 139-147.

### SUMMARY OF INVENTION

### TECHNICAL PROBLEM

As mentioned above, many antiparasitic agents are used against respective parasitic infections, but they involve various problems such as limited activity spectrum, which constitutes a serious issue together with the emergence of the resistant strains as noted above. Therefore, it is imperative to develop a novel antiparasitic agent which has efficacy against a wide range of parasites as well as high safety.

The objective of the present invention is to provide an antiparasitic agent comprising a novel active ingredient, especially a pharmaceutical composition as an antiparasitic agent having both antihelminthic and antiprotozoal activities.

### SOLUTION TO PROBLEM

In order to achieve the above-noted objective, the present inventors focused on at least one effective active substance produced by *Helminthosporium siccans* which is a filamentous fungus, i.e. monocyclofarnesol (e.g., a siccanin or a chemically synthesized compound or modified derivative thereof).

In order to obtain a wide spectrum of effects against helminths and protozoa, the present inventors started their studies, focusing on respiratory chain complex II which is a common target in these parasites.

Respiratory chain complex II is one of the enzymes that constitutes a mitochondrial respiratory chain and exists in two types: succinate-quinone oxidoreductase (SQR) which contributes to ATP production in a cell by utilizing the reducing ability of succinate, and quinol-fumarate oxidoreductase (QFR) which performs a reverse reaction thereof. As with other respiratory chain enzymes, the subunit structure of complex II differs across organism species (Non-Patent Document 8), but is generally composed of four parts: flavoprotein subunit (Fp), iron-sulfur protein subunit (Ip), and two hydrophobic cytochrome b subunits (CybL and CybS) (Non-Patent Document 9). Fp is a subunit that comprises succinate- and fumarate-binding sites, with FAD being covalently linked as an electron receptor. Ip is a hydrophilic subunit containing three types of iron-sulfur clusters and plays a role in mediating electron transfer between FAD and quinone. Membrane-anchoring CybL and CybS form quinone-binding domain together with part of Ip subunit. In addition to complex II which is composed of these four subunits, three more complexes have been identified: complex II in part of Gram-negative bacteria and the like which is composed of three subunits with CybL fused to CybS (Non-Patent Document 10); further, complex II in plants which has eight subunits in total (Non-Patent Document 11); and complex II in trypanosomatids which is composed of 12 subunits (Non-Patent Document 12).

Complex II as a target of antiparasitic agents is an enzyme which directly connects a TCA cycle and a respiratory chain, and it is also one of the most important drug targets. Compounds such as carboxin, flutolanil, and fluopyram are known to exhibit high inhibitory effects chiefly against fungal respiratory chain complex II, and some of these compounds have been commercialized as agrochemicals in recent years. Furthermore, several helminths including roundworms use QFR for anaerobic energy metabolism in order to adapt to low oxygen environments in hosts, and respiratory chain complex II plays a major role in their survival. Among the aforementioned fungal complex II inhibitors, flutolanil is known to inhibit the pig roundworm *Ascaris suum* complex II with 800-fold selectivity over pig complex II (Non-Patent Document 8). Respiratory chain complexes are also important drug targets in trypanosomatids, and it is suggested that respiratory chain enzymes are acted upon by compounds that exhibit antiprotozoal effect, especially in leishmaniasis, as exemplified by miltefosine (respiratory chain complex IV (Non-Patent Document 13)), tafenoquine (respiratory chain complex III (Non-Patent Document 14)), and sitamaquine (respiratory chain complex II (Non-Patent Document 15)). In particular, trypanosomal complex II has a unique structure consisting of 12 subunits and is known to show a greatly different inhibitor susceptibility than mammalian complex II. Since this subunit structure is preserved in the genomes of *T. cruzi*, *T. brucei*, and pathogenic leishmania, the enzyme may be a potential drug target in all of the three major types of trypanosomiasis. However, complex II which is composed of 12 subunits exhibits lower sensitivity to the known complex II inhibitors than mammalian complex II, and no potent selective inhibitors have been found. Although the above-noted antileishmanial compound sitamaquine selectively inhibits fumarate-dependent oxygen consumption in mitochondria, it exhibits only a weak inhibitory effect against the enzyme itself (Non-Patent Document 15). Furthermore, there have been found no therapeutics for African sleeping sickness and Chagas disease that target respiratory chain complex enzymes.

In the present invention, siccanin, a compound which had been known as an antifungal agent, was observed for its selective inhibitory effect against nematode and trypanosomal respiratory chain complex II, as well as for its cell growth inhibitory effect.

Siccanin is a compound isolated from *Helminthosporium siccans* in 1962 (Non-Patent Document 16) and forms a cys, syn, cis-fused alicyclic structure composed of two cyclohexane rings, one oxane ring, and one benzene ring (non-patent document 17). Siccanin has antifungal activity and is known to exhibit a growth inhibitory effect on infectious fungi such as *Trychophyton spp., Epidermophytonfloccosum,* and *Microsporium gypseum* (MIC: 0.6-6.3 µg/ml (Non-Patent Document 18)). Albino guinea pigs (body weight 350 g, male) were used.

In the *T. mentagrophytes* infection experiment, a glycol salicylate ointment containing 1% siccanin was applied to an affected area 3-8 days after the infection, whereby the skin of 85-100% of the subjects was completely cured ((N=20) Non-Patent Document 19). After clinical studies, siccanin was commercialized as a skin infection therapeutic agent and had been sold by Daiichi Sankyo, Co., Ltd. (formerly Sankyo Co., Ltd.) until 2005. Although siccanin does not inhibit NADH- or ascorbate-dependant oxygen consumption in *T. mentagrophytes* mitochondria, it potently inhibits the activity of respiratory chain complex II (IC₅₀ = 87 nM (Non-Patent Document 20)).

In addition, a unique species-selective inhibitory effect of siccanin has been newly discovered in recent years (Non-Patent Document 21). Siccanin exhibits the inhibitory effect against *Pseudomonas aeruginosa* complex II (IC₅₀ = 0.87 µM) and rat complex II (IC₅₀ = 9.3 µM), but not against *Escherichia coli* complex II (IC₅₀ = 268 µM) or pig complex II (IC₅₀ = 861 µM). By a kinetic study using the membrane fraction of Pseudomonas aeruginosa, the inhibition mode of siccanin was presumed to be mixed inhibition against quinone, and the site of action for complex II was estimated to be the quinone-binding domain.

The present invention has been accomplished based on the above-stated findings.

More specifically, the present invention provides an antiparasitic agent or antiparasitic composition comprising at least one compound selected from a siccanin and modified derivatives thereof as an active ingredient.

The aforesaid antiparasitic agent or antiparasitic composition can be used as a pharmaceutical for humans, pharmaceutical for animals, agrochemical, or pharmaceutical for fish.

### ADVANTAGEOUS EFFECTS OF INVENTION

The antiparasitic agent of the present invention is extremely useful as a prophylactic and therapeutic agent for parasitic infections in a wide range of organisms such as humans, animals other than humans, and fish. Furthermore, the antiparasitic agent of the present invention can be used as an antiparasitic agent in agricultural or horticultural applications.

### BRIEF DESCRIPTION OF DRAWINGS

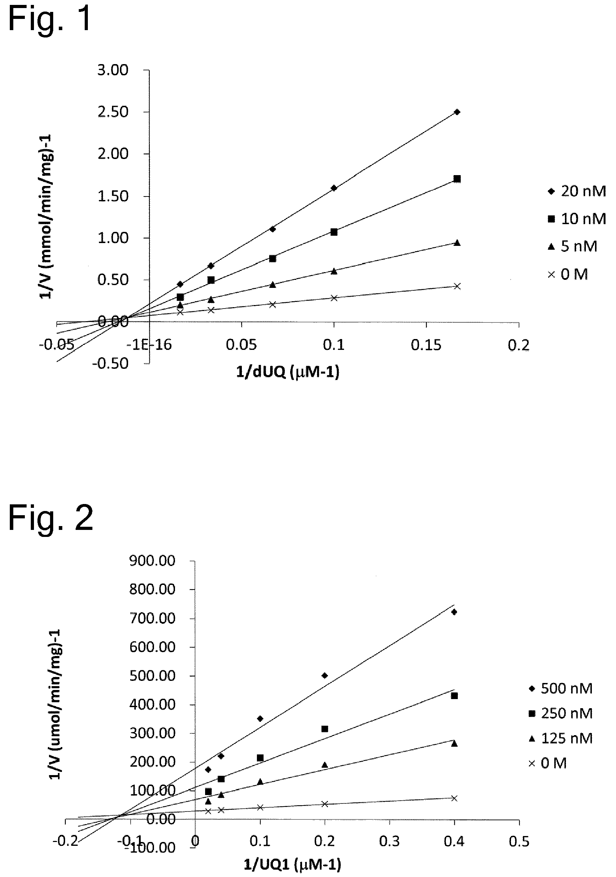

Fig. 1 is a graph which represents the inhibition pattern of siccanin against *A. suum* respiratory chain complex II. The Lineweaver plot shows how siccanin inhibits the *A. suum* SQR activity for decylubiquinone. The concentrations of siccanin are 20 nM, 10 nM, 5 nM, and 0 M, and according to the position of the point at which the four lines intersect, the inhibition mechanism is estimated to be mixed inhibition (Kᵢ₁ = 3.0 nM, Kᵢ₂ = 8.7 nM).
Fig. 2 is a graph which represents the inhibition pattern of siccanin against *L. tarentolae* respiratory chain complex II. The Lineweaver plot shows how siccanin inhibits the *L. tarentolae* SQR activity for ubiquinone 1. The concentrations of siccanin are 500 nM, 250 nM, 125 nM, and 0 M, and according to the position of the point at which the four lines intersect, the inhibition mechanism is estimated to be mixed inhibition (Kᵢ₁ = 39 nM, Kᵢ₂ = 102 nM).

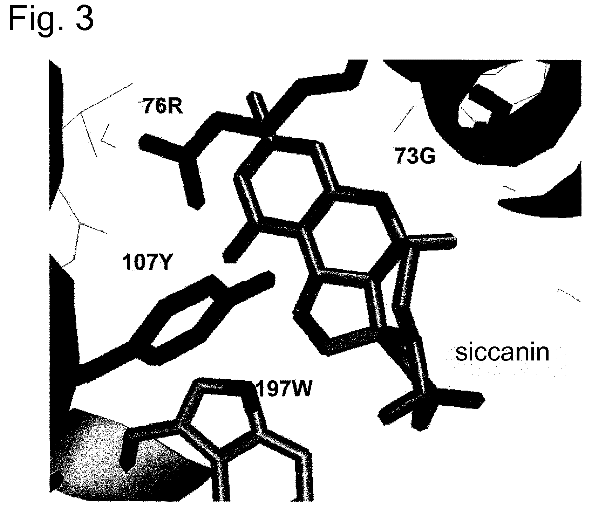

Fig. 3 is a schematic representation which illustrates a model for the binding of siccanin and *A. suum* complex II. This binding model was *generated in silico* based on flutolanil- *A. suum* complex II cocrystal (3VR9). Hydrogen bonds were presumed to form with Trp197 of the subunit and Tyr107 of CybS subunit, and, further, the π-electron carrier benzene ring structure was found to exist near Arg76 residue of CybL subunit. Moreover, the distance between Gly73 residue of CybL subunit and the methyl group at position 7 of siccanin is 2.9Å, which suggests a possibility for the formation of hydrophobic interaction.

### DESCRIPTION OF EMBODIMENTS

The present invention encompasses the disclosure of an antiparasitic agent or a method of eliminating parasites as noted below.
[1] An antiparasitic agent comprising at least one compound selected from a siccanin or modified derivatives thereof as an active ingredient.
[2] The antiparasitic agent of [1] wherein the siccanin is a substance produced by *Helminthosporium siccans* which is a filamentous fungus.
[3] The antiparasitic agent of [1] wherein the siccanin is a chemically synthesized compound.
[4] The antiparasitic agent according to any one of [1] to [3], which is used for pharmaceuticals purposes.
[5] The antiparasitic agent according to any one of [1] to [4], which is used as a veterinary pharmaceutical.
[6] The antiparasitic agent according to any one of [1] to [3], which is used as an agrochemical.
[7] The antiparasitic agent according to any one of [1] to [4], which is used as a pharmaceutical for fish.
[8] A method of eliminating a parasite comprising a step of administering the antiparasitic agent according to any one of [1] to [7] to a host that carries the parasite.

The siccanin used in the present invention is a white to pale yellow crystal produced by *Helminthosporium siccans* as represented by the following formula.

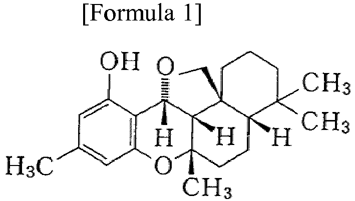

Siccanin has been used as an antifungal antibiotic for the treatment of dermatophytosis, such as trichophytia pompholyciformis, marginated eczema, and tinea corporis, in the form of topical agent, but it has been totally unknown that the compound is useful as an antiparasitic agent.

As used herein, the terms "antiparasitic agent" and "antiparasitic composition" have the same meaning.

As used herein, the term "siccanin or a chemically synthesized compound or a modified derivative thereof" refers to a natural siccanin obtained from *Helminthosporium siccans*, a chemically synthesized siccanin, or any derivative of the natural siccanin or chemically synthesized siccanin (including salts of such siccanin). The siccanin used in the present invention may be a natural or synthesized siccanin. Examples of such derivatives of the siccanin include salts of the siccanin, e.g., sodium salt, potassium salt, calcium salt, magnesium salt, and ammonium salt of the siccanin, as well as esters of the siccanin, e.g., esters of siccanin with acetic acid, propionic acid, butyric acid, acrylic acid, or benzoic acid.

As used herein, the term "siccanin or modified derivatives thereof" has the same meaning as the above-noted "siccanin or a chemically synthesized compound or modified derivative thereof."

As used herein, the term "siccanin" encompasses natural siccanins and chemically synthesized siccanins.

As used herein, "the compound of the present invention" refers to siccanin or a chemically synthesized compound or modified derivative thereof.

The present invention also provides an antiparasitic pharmaceutical composition comprising the compound of the present invention or a salt thereof as an active ingredient, which composition has both antihelminthic and antiprotozoal activities. The compound of the present invention is characterized by having antihelminthic and antiprotozoal activities when the compound is used as a single agent. Accordingly, the aforesaid pharmaceutical composition is preferably such that only the compound of the present invention or a salt thereof is contained as an active ingredient. The aforesaid pharmaceutical composition may further contain other antiparasitic agent(s) as active ingredient(s). Examples of such other antiparasitic agent(s) include but are not limited to: levamisole, pyrantel, albendazole and mebendazole (benzimidazoles-based drugs), as well as avermectin and milbemycin (macrocyclic lactons) as therapeutic agents for helminthiasis; chloroquine, mefloquine, sulfaredoxin, and artemisinin as antimalarial agents; benznidazole, suramin, melarsoprol, nifurtimox, and eflornithine as therapeutic agents for African sleeping sickness; benznidazole and nifurtimox as therapeutic agents for Chagas disease; Glucantime, Pentaptom, amphotericin B, miltefosine, and paromomycin as therapeutic agents for leishmaniasis.

In the use of the compound of the present invention or a salt thereof as a pharmaceutical agent, various dosage forms can be adopted depending on specific prophylactic or therapeutic purposes. Exemplary dosage forms include: oral solid formulations (e.g., powders, fine powders, granules, tablets, coated tablets, and capsules); oral liquid formulations (e.g., dry syrups, syrups, internal liquid agents, elixirs); injections (e.g., subcutaneous, intramuscular, and intravenous injections); and skin liniments that may be used depending on the specific kinds of parasites. These pharmaceutical agents may comprise, as appropriate, a pharmaceutically acceptable excipient, a carrier, or the like in addition to the active ingredient.

In the case of preparing an oral solid formulation, an excipient and, as necessary, a binding agent, a disintegrant, a lubricant, a colorant, and a flavoring agent or an aroma corrigent and the like are added to the compound of the present invention and, thereafter, a conventional method may be applied to produce tablets, coated tablets, granules, powders, capsules and the like. Such an additive may be that which is commonly used in the art and examples include: an excipient such as corn starch, lactose, sucrose, sodium chloride, mannitol, sorbitol, glucose, starch, calcium carbonate, kaolin, microcrystalline cellulose, and silicic acid; a binding agent such as water, ethanol, gum arabic, tragacanth, propanol, simple syrup, glucose solution, starch solution, gelatin solution, carboxymethylcellulose, hydroxypropylcellulose, gelatin, hydroxypropyl starch, methylcellulose, ethylcellulose, shellac, calcium phosphate, polyvinyl alcohol, polyvinyl ether, and polyvinylpyrrolidone; a disintegrant such as gelatin powder, crystalline cellulose, dry starch, sodium alginate, pectin, powdered agar, carboxymethylcellulose, sodium hydrogencarbonate, calcium carbonate, calcium citrate, sodium lauryl sulfate, stearic acid monoglyceride, and lactose; lubricants such as silica, purified talc, stearates, sodium borate and polyethylene glycol; a coloring agent such as those which are approved as an additive including titanium oxide and iron oxide; a flavoring agent or an aroma corrigent such as sucrose, bitter orange peel, citric acid, and tartaric acid.

In the case of preparing oral liquid formulations, a flavoring agent, a buffer, a stabilizer, an aroma corrigent and the like are added to the compound of the present invention and then a conventional method may be applied to produce internal liquid agents, syrups, elixirs and the like. In this case, a flavoring agent and an aroma corrigent may be those mentioned above, and examples of a buffer include sodium citrate while exemplary stabilizers include tragacanth, gum arabic, and gelatin.

In the case of preparing injections, a pH adjusting agent, a buffer, a stabilizer, an isotonic agent, a local anesthetic and the like are added to the compound of the present invention and then a conventional method may be applied to produce subcutaneous, intramuscular, and intravenous injections. Exemplary pH adjusting agents and buffers that may be used in this case include sodium citrate, sodium acetate, sodium phosphate. Exemplary stabilizers include sodium pyrosulfite, EDTA, thioglycolic acid, and thio lactic acid. Exemplary local anesthetics include procaine hydrochloride and lidocaine hydrochloride. Exemplary isotonic agents may be exemplified by sodium chloride and glucose.

Examples of the skin liniment include an ointment, a cream, a gel, a poultice, a plaster, and a patche. In the case of use as an ointment, a creams, or a gel, a grease base, an emulsion base, and the like are used as carriers. Examples of the grease base include oils and fats, a wax, hydrocarbon, fatty acid, alcohol, alkyl glyceryl ether, ester, silicone oil, fluorine oil, and polyhydric alcohol. As oils and fats, vegetable oils and fats as well as animal oils and fats are widely used. Exemplary vegetable oils include olive oil, safflower oil, persic oil, kukui nut oil, wheat germ oil, rice bran oil, rice germ oil, evening primrose oil, high oleic sunflower oil, macadamia nut oil, and meadowfoam oil, and exemplary animal oils include beef tallow, hardened oil, and egg yolk fatty oil.

A Wax contains esters of higher fatty acids and higher alcohols (wax esters) as a major component. The number of carbon atoms that constitute the esters widely ranges from C12 to C34. Exemplary animal waxes include lanolin, spermaceti, bees wax, shellac wax, and liquid orange roughy oil. Exemplary vegetable waxes include carnauba wax, candelilla wax, and liquid jojoba oil.

Examples of the hydrocarbon include chain hydrocarbons, liquid paraffins which are mixtures of various hydrocarbons, branched paraffins, solid paraffins, and petrolatum.

Fatty acids are classified into natural fatty acids and synthesized fatty acids. Examples of the fatty acid include lauric acid, myristic acid, palmitic acid, stearic acid, behenic acid, oleic acid, isostearic acid, 12-hydroxystearic acid, and undecylic acid.

Example alcohols include higher alcohols having carbon numbers of about C8 to about C32, i.e., caprylyl alcohol, capryl alcohol, lauryl alcohol, myristyl alcohol, cetyl alcohol, stearyl alcohol, oleyl alcohol, behenyl alcohol, ceryl alcohol, and lacceryl alcohol. Furthermore, vegetable and animal sterols are also included.

Exemplary alkyl glyceryl ethers include batyl alcohol, chimyl alcohol, selachyl alcohol, and isostearyl glyceryl ether.

An esters is a compounds obtained by dehydration reaction of fatty acid and alcohol. Exemplary esters of linear fatty acid and lower alcohol include ethyl oleate, isopropyl myristate, butyl stearate, and exemplary esters of linear fatty acid and linear higher alcohol include cetyl palmitate and myristyl myristate. Exemplary esters of linear fatty acid and branched higher alcohol include octyldodecyl myristate and octyldodecyl oleate, and exemplary esters of linear fatty acid and polyhydric alcohol include medium-chain triglycerides. Exemplary esters of branched fatty acid and lower alcohols include isopropyl isostearate and butyl isostearate, and exemplary esters of branched fatty acid and linear higher alcohol include cetyl 2-ethylhexanoate and stearyl 2-ethylhexanoate. Examples of esters of branched fatty acid and linear higher alcohol as well as esters of branched fatty acid and branched higher alcohol include isocetyl isostearate, octyldodecyl dimethyloctanoate, and the like. Aside from these esters, myristyl lactate, trioctyldodecyl citrate, and diisostearyl malate may be given as exemplary esters of hydroxycarboxylic acid and alcohol.

Examples of silicone oils include dimethyl silicone oil, methylphenyl silicone oil, cyclic dimethyl silicone oil, methylhydrogen silicone oil, and modified silicone oils, and examples of fluorine oils include perfluoropolyether.

Exemplary polyhydric alcohols include ethylene glycol, diethylene glycol, triethylene glycol, polyethylene glycol, propylene glycol, dipropylene glycol, polypropylene glycol, glycerin, diglycerin, polyglycerin, 3-methyl-1,3-butanediol, and 1,3-butylene glycol.

The amount of the compound of the present invention to be incorporated in each of the aforesaid unit dosage forms varies depending on, for example, the condition of a patient who it should be applied to and its dosage form, and in general, it preferably ranges from about 1 to about 1200 mg in oral formulation, from about 0.1 to about 500 mg in injection, and from about 1% to about 5% in skin liniment, per unit dosage form. Furthermore, the daily dosage of a pharmaceutical which is formulated in each of the aforesaid dosage forms varies depending on, for example, the condition, body weight, age, or sex of a patient and cannot generally be determined but the daily dosage for an adult may usually range from about 0.1 to about 5,000 mg, preferably, from 1 to 1200 mg, and the pharmaceutical is preferably administered in one dose or in two to four divided doses per day.

### EXAMPLES

Examples are given below to illustrate the present invention in more detail, but they are not intended to limit the scope of the present invention.

### [Example 1]

### [Inhibitory Effect of Siccanin against Parasite Respiratory Chain Complex II]

Measurements were made of the inhibitory activities of siccanin against the respiratory chain complex II of the following parasites: the pig roundworm: *A.suum*; the dog filarial: *Dirofilaria immitis*; *Haemonchus contortus*; *Anisakis simplex*; the Chagas disease pathogen: *T. cruzi*; a subspecies of the African trypanosomiasis pathogen: *Trypanosoma brucei brucei*; nonpathogenic protozoan in the genus leishmania of the family *Trypanosomatidae*: *Leishmania tarentolae*,; and tropical malarial protozoan: *Plasmodium falciparum*, (Table 1). The potency of inhibition was evaluated by the concentration at which 50% of the enzymatic activity was inhibited (IC₅₀).

First, the inhibitory effect of siccanin was measured using *A. suum* imago complex II and its IC₅₀ was found to be 5.7 nM. As compared with the IC₅₀ of siccanin against pig complex II (as an indication of siccanin's adverse effects on human and other larger mammal-derived complex II), the selectivity of siccanin was estimated to be 180,000-fold greater than that for *A. suum* complex II. This value corresponds to about 20-fold greater selectivity than the selectivity of flutolanil, previously known as a selective inhibitor against *A. suum* complex II (Non-Patent Document 8), and it is also the greatest of the values presented by the reported inhibitors against roundworm complex II.

Siccanin showed IC₅₀ values of 13.8 nM, 18.2 nM, and 11.2 nM against *D. immiti*, *H. contortus*, and *A. simplex*, respectively, and its selectivity was respectively 62391-fold, 47308-fold, and 76875-fold over pig complex II. Siccanin also showed IC₅₀ values of 1200 nM, 396 nM, and 190 nM against three kinds of trypanosomatids, *T.cruzi*, *T. brucei*, and *L. tarentolae*, respectively, with its selectivity being 718-fold, 3189-fold, and 4532-fold over pig complex II. Furthermore, siccanin inhibited the complex II of the malarial protozoa *P. falciparum* at the IC₅₀ of 98 nm. Siccanin as elucidated in this study is the only compound that has been reported to date as selectively inhibiting trypanosomatid complex II over mammal complex II. The existing therapeutic agents for trypanosomiasis, especially for Chagas disease, cause serious adverse effects, which is a major problem in realizing continued medication, so it has been strongly desired to develop an alternative therapeutic agent. Siccanin, as reported herein, inhibited complex II of the pathogens of the three major types of trypanosomiasis, *T. cruzi*, *T. brucei*, and *Leishmania spp.,* with extremely high selectivities over complex II of a larger mammal (pig), thus demonstrating that siccanin has great potential to act as an anti-trypanosomiasis agent which is highly desirable in terms of reduced adverse effects.

[Table 1]

**Table 1 : The species-selective inhibitory effect of siccanin against respiratory chain complex II activity.**

| Biological species | SQR IC₅₀ (nM) | Selectivity |
|---|---|---|
| Pig^{*1} | 861000 | 1 |
| *A. suum* | 5.7 | 183191 |
| *D. immitis* | 13.8 | 62391 |
| *H. contortus* | 18.2 | 47308 |
| *A. simplex* | 11.2 | 76875 |
| *L. tarentolae* | 190 | 4532 |
| *T. cruzi* | 1200 | 718 |
| *T. b. brucei* | 370 | 3189 |
| *Plasmodium falciparum* | 98 | 8675 |

| | | |
|---|---|---|
| The inhibitory effects of siccanin against succinate-quinoe oxidation-reduction activity were calculated by enzymatic activity measurements for the pig and the following parasites: the pig roundworm: *A. suum*; the dog filaria: *D. immitis*; *H. contortus*; *A. simple*; a protozoan in the genus leishmania of the family nonpathogenic *Trypanosomatidae*: *L. tarentolae*; Chagas disease pathogen: *T. cruzi*; the Nagana disease pathogen: *T. b. brucei*; and the tropical malarial protozoan: *P. falciparum* (n=3). IC₅₀ represents the concentration at which siccanin inhibited 50% of the enzymatic activity, and selectivity represents the ratio of each biological species IC₅₀ to pig IC₅₀. *1 The value of pig SQR activity is cited from literature (Mogi et al., 2009, J Biochem.) | | |

### [Example 2]

### [The inhibitory mechanism of siccanin against nematode and trypanosomatid complex II]

In order to estimate the inhibitory mechanism of siccanin against complex II, the inhibitory pattern was determined by Lineweaver-Burk plot using succinate and quinone as the substrates. As samples for measurement, nematode complex II from *A. suum* and complex II from *L. tarentolae*, which is an easy-to-handle trypanosomatid, were used. First, the inhibitory pattern of siccanin against *A. suum* respiratory chain complex II was mixed inhibition for quinone (Kᵢ₁ = 5.2 nM, Kᵢ₂ = 9.9 nM) (Figure 1), whereas it was non-competitive inhibition for succinate. As for *L. tarentolae* complex II, siccanin exhibited mixed inhibition for quinone, too (*L*. *tarentolae* : Kᵢ₁ = 39 nM, Kᵢ₂ = 102 nM) (Figure 2). In either case, Kᵢ(Kᵢ₁) of siccanin against complex II alone was lower than Kᵢ(Kᵢ₂) against the complex of respiratory chain complex II and ubiquinone, which suggests that the affinity of siccanin for complex II lowered due to the binding of ubiquinone. Furthermore, these enzymes were measured for the activity in succinate dehydrogenase enzyme (SDH) reaction using artificial substrates PMS and MTT in place of quinone but no inhibitory effects of the enzymatic activity were observed even in the presence of 50 µM siccanin. The SDH activity is catalyzed by only subunits Fp and Ip of complex II and the quinone-binding domain is not essential for the reaction. Based on these results, the binding domain of siccanin to complex II is presumably located around the quinone-binding domain.

### [Example 3]

### [Structural model for the complex of A. suum complex II and siccanin]

As of today, no report had been made on the analysis of a 3D structure for the complex of siccanin and respiratory chain complex II, so the binding pattern of siccanin is yet to be elucidated directly at the atomic level. Instead, focusing on the fact that siccanin is an inhibitor that targets the quinone-binding domain of complex II, the present inventors generated a model for the binding mode of siccanin based on the cocrystals of *A. suum* complex II and quinone inhibitors, of which a pdb file had already been registered (Figure 3). As a result of docking *calculation in silico*, the cocrystal with flutolanil provided the best-fitting coordinates for the binding compound and siccanin (Non-Patent Documents 22 and 23).

In the complex of *A. suum* complex II and flutolanil, the binding site for the inhibitor is known to be composed of Trp197 of Ip subunit, Try107 of CybS subunit, and Trp69 and Arg76 of CybL subunit. The selectivity of flutolanil comes from the roundworm-specific π-interaction at the Trp69 and Arg76 residues of CybL subunit (Non-Patent Documents 8 and 23).

In the docking model, the binding mode of *A. suum* complex II with siccanin was similar to that of binding with flutolanil, and hydrogen bonds were presumed to exist between Trp197 of Ip subunit and Try107 of CybS subunit, and further a benzene ring as a π-electron carrier was found to exist near the Arg76 residue of CybL subunit. On the other hand, there were no constituent atoms of siccanin near Trp69 of CybL subunit and no π-interaction was predicted. As for siccanin-specific binding, hydrophobic interaction was predicted to exist between the methyl group at position 7 of siccanin and the Gly73 residue of CybL subunit. In pig complex II (Non-Patent Document 24), this glycine is replaced by isoleucine having a larger side chain, so a possibility is suggested that the steric hindrance at this site may have caused the low susceptibility of siccanin to pig complex II.

### [Example 4]

### [The antimicrofilarial effect of siccanin]

As shown in Table 1, siccanin inhibited *D. immitis* complex II at the IC₅₀ of 13.8 nM, as against *A. suum* complex II. Furthermore, the anti-nematode effect of siccanin was evaluated by using the microfilariae of *D. immitis.* As a result, 5 µM siccanin exhibited the same effect as 5 µM ivermectin that was used as a control, and 50 µM siccanin inhibited the motility of the microfilariae much more potently than ivermectin (Table 2).

[Table 2]

**Table 2 The anti-nematode effect of siccanin against D. immitis at the microfilaria larval stage. Microfilariae in the blood of an infected dog were purified by PD10 column, and 100 microfilariae were transferred into each well and the killing effect of siccanin was measured at 24 and 48 hours after the transfer. The control was ivermectin currently in clinical use. As shown in the table, 5 µM siccanin exerted a comparable effect to ivermectin, and 50 µM siccanin exerted a greater killing effect than ivermectin.**

| Motility (%) | | | | | |
|---|---|---|---|---|---|
| | DMSO | Siccanin | | Ivermectin | |
| | | 5µM | 50µM | 5µM | 50µM |
| 0 hr | 100 | 100 | 25 | 100 | 100 |
| 24 hr | 100 | 75 | 0 | 75 | 50 |
| 48 hr | 100 | 50 | 0 | 50 | 50 |

### [Example 5]

### [The antiprotozoal effect of siccanin]

Siccanin inhibits trypanosomatid and plasmodia complex II with IC₅₀ values on the nM order. Based on this fact, the present inventors further measured the impact of siccanin on the proliferation of parasites (Table 3). The species of the organisms used in the present study were: nonpathogenic leishmanial protozoan *L. tarentolae* promastigote (insect form), cutaneous leishmaniasis pathogen *L. major* promastigote (insect form), visceral leishmaniasis pathogen *L. donovani* promastigote (insect form), related species of African sleeping sickness pathogen *T. b. brucei* bloodstream form, Chagas disease pathogen *T. cruzi* amastigote (cell form), and plasmodia *P. falciparum* erythrocytic stage (Table 3). *T. b. brucei* bloodstream form and *T. cruzi* amastigote correspond to the stages found in mammalian hosts and can be used as target forms in drug treatments for African trypanosomiasis and Chagas disease.

In the presence of 0.05 to 50 µM siccanin, the proliferation of *L. tarentolae* promastigote, *L. donovani* promastigote, *L. major promastigote*, and *T. brucei* bloodstream form was measured by Alamar Blue method. The calculated IC₅₀ values of siccanin were 4.4 µM for *L. tarentolae*, 700 nM for *L. donovani*, 13 µM for *L. mayor*, and 8.3 µM for *T. b. brucei* (Table 3). The growth inhibitory effect of siccanin on *T. cruzi* was evaluated by counting the *T. cruzi* amastigote population that infected mouse cells. As compared with DMSO control, the number of the protozoa decreased by 50% in the presence of 10 µM siccanin. In addition, siccanin exhibited a weak inhibitory effect on the proliferation of *P. falsiparum.* The IC₅₀ value as calculated by LDH assay was 15.53 µM.

[Table 3]

**Table 3. The in vitro protozoan growth inhibition by siccanin.**

| Biological species | Celluler IC50 (µM) |
|---|---|
| *L. tarentolae* promastigote | 4.4 |
| *L. donovani* promastigote | 0.7 |
| *L. major* promastigote | 13 |
| *T. b. brucei* bloodstream form | 8.3 |
| *T. cruzi* amastigote | 10 |
| *P. falciparum* erythrocytic stage | 16 |

Calculation was made of the cell growth inhibitory effect of siccanin on *L. tarentolae* promastigote, the visceral leishmaniasis pathogen *L. donovani* promastigote, the cutaneous leishmaniasis pathogen *L. major* promastigote, *T. cruzi*, *T. b. brucei*, and *P. falciparum.* Cellular IC₅₀ refers to the concentrations of siccanin at which the growth of respective cells was inhibited by 50%.

### [Example 6]

### [In vitro cell growth inhibitory effect of siccanin as compared with existing therapeutic agents for trypanosomiasis]

Amphotericin B administered by intravenous injection exerts the strongest antiprotozoal action among the therapeutic agents currently used as treatment agents for leishmaniasis, and its IC₅₀ is about 12 nM for *L. donovani* promastigote and about 43 nM for *L. major* promastigote (Non-Patent Document 25). However, injectable drugs including amphotericin B have strong adverse effects and need to be replaced by alternative drugs. It has been reported that miltefosine, which is the only oral medication that is currently available among the therapeutic agents for leishmaniasis, has IC₅₀ values of ∼450 nM for *L. donovani* promastigote and about 13.1 µM for *L. major* promastigote (Non-Patent Document 25). Resistant strains have been reported against each of these agents and therapy using combination drugs is currently used widely. It is known that benznidazole and nifurtimox that are used as therapeutic agents for Chagas disease exhibit the inhibitory effects against *T*. *cruzi* amastigote with *in vitro* IC₅₀ values of about 22 µM and about 3.5 µM, respectively (Non-Patent Document 26). These drugs are administered via intravenous injection and have particularly strong adverse effects. Suramin, pentamidine, and melarsoprol, which are major therapeutic agents for African sleeping sickness, have *in vitro* IC₅₀ values of 15 nM, 0.6 nM, and 7 nM, respectively, for *T. brucei rhodensiense* bloodstream form, and 349 nM, 0.9 nM, and 4 nM, respectively, for *T. brucei gambiense* bloodstream form (Non-Patent Document 27).

The IC₅₀ of siccanin against the pathogenic leishmanial protozoa assayed herein was several tens of times greater than that of amphotericin B and comparable to that of miltefosine. The IC₅₀ of siccanin against *T. cruzi* amastigote was twice the value of nifurtimox, and about half of the value of pentamidine. That is, siccanin exhibits the antiprotozoal effects no less inferior to those of the existing therapeutic agents for leishmaniasis and Chagas disease at the *in vitro* stage. In contrast, the IC₅₀ value of siccanin against *T. brucei* is greater than those of the existing suramin, pentamidine, and melarsoprol. However, siccanin exhibits higher selectivity for the target enzyme than these compounds having strong adverse effects, and, thus, has a potential for becoming a new drug having less adverse effects than the existing compounds. In addition, siccanin has a complex skeleton comprising four carbocyclic rings linked together, a structure greatly different from those of antimony compounds, phosphatidylcholine compounds, azole compounds, or aminoquinoline compounds, to which the existing trypanosomal drugs belong. Considering the strains that are resistant to the existing drugs, the unique structure of siccanin could be a great advantage for drug treatments.

### Conclusion

As clearly shown in the above Examples, siccanin which had been recognized as an antifungal agent was first revealed to have the nature of an antiparasitic agent. Siccanin is an extremely selective inhibitor of parasite respiratory chain complex II and potently inhibits the enzymes in nematodes and trypanosomatids, whereas it is almost ineffective in mammals. In addition, siccanin exhibited proliferation inhibitory effects against a variety of pathogens of parasitic diseases such as African trypanosomiasis, Chagas disease, leishmaniasis, and falciparum malaria, and, further, siccanin exhibited growth inhibitory effects against nematodes. Thus, siccanin and modified derivatives thereof are compounds that can be unprecedented antiparasitic agents which have low adverse effects on mammals and feature an effective spectrum covering a plurality of protozoan infections and parasitic nematode infections.

Formulation examples of the antiparasitic agent of the present invention are shown below.

### Formulation Example 1 Tablet

**[Table 4]**

| | |
|---|---|
| Siccanin | 50.0 mg |
| Mannitol | 20.3 mg |
| Hydroxypropylcellulose | 2.5 mg |
| Crystalline cellulose | 10.0 mg |
| Corn starch | 10.0 mg |
| Calcium carboxymethyl cellulose | 5.0 mg |
| Talc | 2.0 mg |
| Magnesium stearate | 0.2 mg |

Tablets each weighing 100 mg was prepared in the usual manner from the above-noted formulation.

### Formulation Example 2 Granule

**[Table 5]**

| | |
|---|---|
| Siccanin | 300 mg |
| Lactose | 540 mg |
| Corn starch | 100 mg |
| Hydroxypropylcellulose | 50 mg |
| Talc | 10 mg |

Granules weighing 1000 mg per pack was prepared in the usual manner from the above-noted formulation.

### Formulation Example 3 Capsule

**[Table 6]**

| | |
|---|---|
| Siccanin | 50 mg |
| Lactose | 15 mg |
| Corn starch | 25 mg |
| Microcrystalline cellulose | 5 mg |
| Magnesium stearate | 1.5 mg |

Capsules each weighing 96.5 mg were prepared in the usual manner from the above-noted formulation.

### Formulation Example 4 Injection

**[Table 7]**

| | |
|---|---|
| Siccanin | 100 mg |
| Sodium chloride | 3.5 mg |
| Injectable distilled water (2 mL/ampule) | q.s. |

An injection was prepared in the usual manner from the above-noted formulation.

### Formulation Example 5 Syrup

**[Table 8]**

| | |
|---|---|
| Siccanin | 200 mg |
| Purified sucrose | 6.0 g |
| Ethyl p-hydroxybenzoate | 5 mg |
| Butyl p-hydroxybenzoate | 5 mg |
| Scent | q.s. |
| Coloring agent | q.s. |
| Purified water | q.s. |

Syrup was prepared in the usual manner from the above-noted formulation.

### Formulation Example 6 Tablet

**[Table 9]**

| | |
|---|---|
| Siccanin | 50 mg |
| Artemether | 20 mg |
| Cyclodextrin | 26 mg |
| Microcrystalline cellulose | 5 mg |
| Hydroxypropylcellulose | 5 mg |
| Talc | 2 mg |
| Magnesium stearate | 2 mg |

Tablets each weighing 120 mg were prepared in the usual manner from the above-noted formulation.

### Formulation Example 7 Ointment

**[Table 10]**

| | |
|---|---|
| Siccanin | 10 g |
| White petrolatum | 200 g |
| Liquid paraffin | 600 g |
| Propylene glycol | 120 g |
| Flavor | q.s. |

A 1% ointment was prepared in the usual manner from the above-noted formulation.

### INDUSTRIAL APPLICABILITY

The present inventors revealed that siccanin conventionally used as an antimicrobial agent (antifungal agent) is effective as a prophylactic and therapeutic agent for parasitic infections in a wide range of organisms such as humans, animals other than humans, and fish.

The present invention provides an antiparasitic agent, or an antiparasitic composition, comprising at least one compound selected from a siccanin and modified derivatives thereof as an active ingredient, and it has extremely marked effects as described above in detail. Furthermore, the antiparasitic agent of the present invention can be used in a wide variety of fields, as a pharmaceutical for humans, a pharmaceutical for animals, an agrochemical, or a pharmaceutical for fish. The present invention will therefore prove to be extremely useful in industries.

## Claims

1. An antiparasitic agent comprising at least one compound selected from a siccanin and modified derivatives thereof.

2. The antiparasitic agent of Claim 1 wherein the siccanin is a substance produced by *Helminthosporium siccans* which is a filamentous fungus.

3. The antiparasitic agent of Claim 1 wherein the siccanin is a chemically synthesized compound.

4. The antiparasitic agent according to any one of Claims 1 to 3 which is used for pharmaceutical purposes.

5. The antiparasitic agent according to any one of Claims 1 to 4, which is used as a veterinary pharmaceutical.

6. The antiparasitic agent according to any one of Claims 1 to 3, which is used as an agrochemical.

7. The antiparasitic agent according to any one of Claims 1 to 4, which is used as a pharmaceutical for fish.
